# EUROPEAN PATENT SPECIFICATION

(11) **EP 0 993 316 B1**
(45) Date of publication and mention of the grant of the patent: **08.12.2004**
(21) Application number: 98931181.6
(22) Date of filing: 26.06.1998
(51) Int. Cl.: A61M 25/01, A61B 17/34

(54) **DEVICE TO FACILITATE THE REMOVAL OF BILE DUCT STONES**
VORRICHTUNG UM DAS ENTFERNEN VON STEINEN AUS DEM GALLENGANG ZU ERLEICHTERN
DISPOSITIF PERMETTANT DE FACILITER L'ELIMINATION DE CALCULS DANS LES CANAUX BILIAIRES

(30) Priority: 30.06.1997 SE 9702516
(43) Date of publication of application: 19.04.2000
(73) Proprietor: Handelsbolaget B & B Läkartjänst, 907 50 Umea (SE)
(72) Inventor: BLIND, Per, Jonas, S-907 50 Umeä (SE)
(74) Representative: Onn, Thorsten
(86) International application number: PCT/SE1998/001260
(87) International publication number: WO 1999/000156

(56) References cited:
- US-A- 5 146 925
- US-A- 5 167 645
- US-A- 5 334 143

## Description

The present invention concerns an arrangement to facilitate the removal of stones from the bile duct of a person.

An arrangement according to the preamble of claim 1 is disclosed in US-A-5 334 143.

The problems of known techniques are exemplified here by referring to the treatment of people with stones in their bile ducts. To avoid drastic surgical measures, that known as percutaneous transhepatic choledochoscopy (PTC) can be employed, which means that a sleeve is arranged through the patients skin and into a bile duct. The introduction of the sleeve is usually carried out by inserting a needle through the skin on the right-hand side of the patient and then on through the liver and into a bile duct. To verify the position of the needle, a contrasting solution is injected and the patient X-rayed. When it has been confirmed that the needle has reached its target bile duct. a thin guide is inserted through the needle. after which the needle is removed. Successively broader sleeves known as dilatation catheters are then introduced step-by-step over the guide to expand the opening through the body. In this way, troublesome over-pressure can be evacuated.

When the desired diameter has been reached, the patient carries the sleeve for two to four weeks so that a proteinaceous channel can form around the sleeve. The sleeve is then removed and the large channel used for the insertion of the instrument chosen for treatment, e.g. a laser, endoscope, etc. The very frail wall of the channel through the body is, however, a problem. If the instrument needs to be repeatedly inserted or if an instrument hits the wall at an oblique angle, there is a great risk that the instrument breaks through the fragile proteinaceous wall, whereby the procedure has to be repeated from the beginning with the insertion of a new needle, etc. This leads to time consuming, expensive and. for the patient, very uncomfortable treatment.

Naturally, the outer sleeve can in theory remain in place during the treatment. This is, however. not a practical alternative as it is desirable that the opening formed in the patient is kept as small as possible.

Further problems arise when the instrument, which can be one or several, is inserted through the channel and further on through the bile ducts. These ducts are curved, not straight. Instruments that are flexible but commonly still relatively firm - which is preferable if they are to be manoeuvred without them bending - tend to scrape their free end against the outer wall of the curve and damage this as they force their way along the curved ducts. This can cause bleeding that can ruin the sight for an endoscope, for example, and make laser treatment impossible.

When treating stones in bile ducts, the stones can be located in different positions in the bile ducts or move during the actual treatment, which makes it necessary to repeatedly move the instrument forwards and backwards in the bile ducts during the treatment. Such movement naturally further increases the risk of damaging the walls.

To evacuate the stones, they must be broken down to small particles of gravel to be conveyed out through the bile opening to the intestine. This opening is commonly about 3 mm, which thus requires that the particles of gravel are smaller. To be able to evacuate even larger particles. measures can be taken to expand the bile opening. This expansion can take place via the introduction of an instrument via the entrance to the intestine, which causes very unpleasant feelings for the patient.

The objective of the present invention is to overcome the disadvantages named above and achieve an arrangement first mentioned above and that has the features specified in the characteristics section of the main claim.

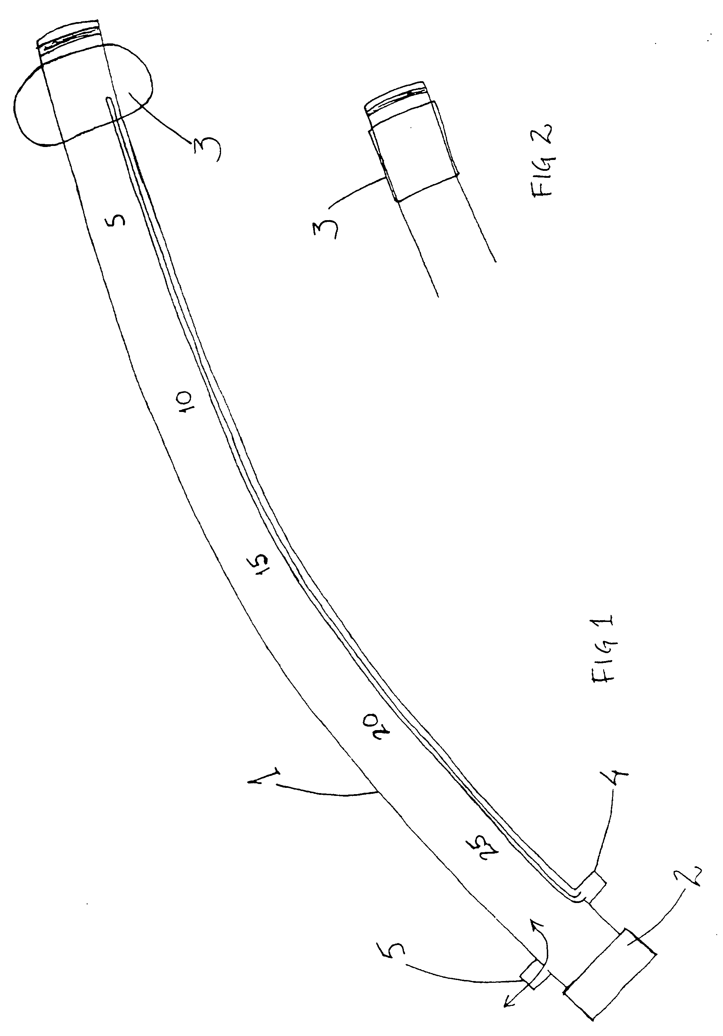

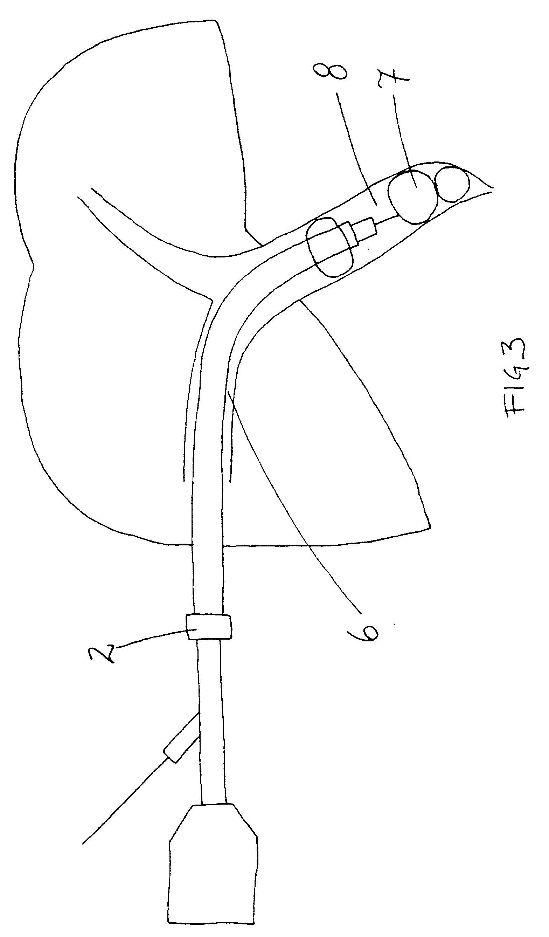

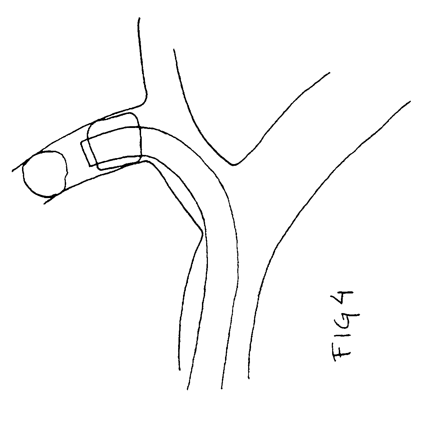

Other objectives and features of the invention will be evident from the following detailed description of one embodiment given with reference to the enclosed drawings, where fig. 1 shows schematically an arrangement according to one embodiment of the present invention, fig. 2 shows a view of a detail of the arrangement according to fig. 1. fig. 3 shows schematically the arrangement according to fig. 1 inserted through a bile duct for treating a stone deep in a duct, and fig. 4 shows schematically the use of an alternative embodiment of the arrangement for treating a stone in a side duct.

One embodiment of the arrangement according to the invention especially adapted to the treatment or sampling of bile ducts is described with reference to fig. 1. The arrangement includes a tube-shaped section 1 in a material that is flexible yet comparatively resistant to torsion. The material of tube-shaped section 1 is flexible so that it can follow the curves when it is inserted through curved ducts. The tube-shaped section 1 should also be resistant to torsion so that it can be manoeuvred, e.g. turned when inserted to direct its end. To minimise the damage to the patient, the tube-shaped section can be used as the final, widest dilatation catheter. It should have a thin a wall as possible to ensure that a specified inner diameter can be achieved with as small an external diameter as possible. The thinness of the wall is nevertheless limited, partly by the said desirability for resistance to torsion, but also by a need for resistance to bending of the material when the arrangement is pushed through the body. Examples of suitable material can be one of the current catheter materials that do not damage the body. The material can be transparent.

It can also be noted here that the arrangement can even be used so that it is inserted through a broader sleeve.

The inner diameter can vary depending on the type of procedure planned. In general, an arrangement is chosen that has the smallest possible inner diameter to accommodate the instrument required for the actual procedure.

The tube-shaped tube section 1 has a front and a rear free end. The rear end is equipped with a cap 2 that has at least one closeable opening through which an instrument can be introduced. This opening seals tightly around the instrument to avoid possible leakage of fluids. The cap is preferably removable so that it can quickly be exchanged if the actual instrument requires a special shape or sealing of the instrument's insertion opening or if the cap needs to be removed for evacuation.

An inflatable balloon-like part 3 is arranged at the front end of tube 1 and directly adjacent to the end. In the present embodiment, balloon 3 extends symmetrically around the tube with an axial extension in the range 10 - 35 mm. The material of the balloon is elastic and in a stretched state is equivalent to several times its non-affected area. The material should preferably be of a type that does not fracture and leave remains in the patient should the balloon be punctured. One example of a suitable material is polyurethane. but other elastic materials can also be used.

The balloon can be connected to a pressure-regulating device (not shown) via its own channel 4 in the tube for regulating the pressure and thereby the balloon's shape and outer diameter. The balloon channel 4 can with advantage be arranged in the tube's casing or it can run in the interior of the sleeve through a tube arranged there. for example. The pressure-regulating device can, for example, comprise a hand pump and a valve or some other means of regulating pressure suitable for a person skilled in the area.

In addition. the front end of the arrangement preferably has a marker that can be seen when X-rayed, e.g. a ring or a wire that restricts the penetration of X-ray beams arranged round the tube. The marker can also be recessed or embedded in the tube part.

Because of its area of intended use. the arrangement according to the present embodiment is about 20 - 40 cm long, depending on the physique of the patient. The tube has measurement markings along its length. The task of these measurement markings is to indicate the distance to the front end of the arrangement and thus the depth of penetration.

In addition, a valve-regulated device 5 intended for draining and thus constituting that known as an irrigation channel is arranged at the rear end of the tube part that, in its working position, is outside of the patient.

Fig. 2 shows the balloon in its initial state where it has essentially the same external diameter as its closest surroundings. This is the starting position the balloon is in while it passes along a dilatation sleeve arranged in the patient.

When the front end has passed the dilatation sleeve and entered a cavity, duct, channel or organ of larger diameter, the balloon can be inflated somewhat to form a cushion-shaped front end of the arrangement with a comparatively larger radius that protects the cavities' walls.

Fig. 3 shows schematically the arrangement according to the present embodiment inserted through the skin and liver and into a bile duct 6. When the front end has entered the bile duct, the patient is X-rayed to verify the position of the front end. Following this, the balloon 3 is inflated a little more to protect the bile duct walls during the continued penetration. Since the bile duct is curved, the tube-shaped front end would strike and shear off the bile duct's wall if it were not for the protection provided by the radius of the balloon. When well-positioned in the bile duct, an endoscope. for example, can be inserted through the opening in cap 2 for inspection. The arrangement can also be used to push stones present in the bile duct through which the arrangement is being inserted in front of it.

Instruments can also be inserted through the tube to push forward stones or, with the help of a grasping device, to directly pick up smaller stones that can pass through the tube without having to be broken down. In the latter case, the balloon can with advantage be inflated further so that it rests against the bile duct wall, whereby it partly avoids the risk that the stone ends up in a position past the end of the tube, partly ensures a firm positioning of the tube prior to and during the procedure, and partly centres the opening of the tube in relation to the duct's walls.

By gathering together the stones 7 in the deep bile duct 8, the treatment to break down the stones can take place efficiently and with high accuracy. This breakdown can be accomplished with for, example, forceps, lithotriptor basket, lasers, electrohydraulic lithotriptor, or ultrasound. The use of a pulsating colour laser or pulsed dye laser introduced through the arrangement is suggested.

When moving or treating the stones, it is advantageous if the operator knows where the stones are located. There is a risk that they can squeeze past the front end of the arrangement thereby escaping being broken down and thus remaining behind as stones or gravel particles after treatment. To avoid this, balloon 3 can, according to the present invention, be inflated further so that it forms a tight seal against the bile duct walls. In this way, the treatment area is limited by the front end, the balloon and the bile duct wall, from which the stones or gravel particles cannot escape.

The gravel particles left after the stones have been broken down can, on completion of treatment, be removed through the bile opening onto the intestine if they are sufficiently small, or preferably through the main channel 1 of the tube. To ensure that all the gravel has been removed, it can be advantageous to flush a liquid through tube 1.

One advantage of flushing out the gravel through tube 1, preferably after the other instruments have been removed, is that the gravel can be removed after a lower degree of breakdown. i.e. the gravel particles can have larger diameters than when they pass out through the bile opening to the intestine. This relationship in turn leads to a reduced time for treatment, since the degree of breakdown largely depends on the time taken for the laser treatment.

The arrangement according to the present invention can even be used for treating a stone or stones entrapped in connecting bile ducts. For this objective, it can be advantageous to use other embodiments of the invention.

In the embodiment shown in fig. 4. the tube 1 has an asymmetric balloon, which means that the balloon can only be inflated within a specified range of circumference around the tube at the front end. This facilitates the front end's chance of passing a corner or a junction. The balloon's non-symmetrical position around the tube makes it possible for the operator to manoeuvre the end into side ducts by turning the tube in combination with, for example, regulating the degree of inflation of the balloon.

As mentioned previously, when the balloon is inflated, gravel is prevented from squeezing past the front end. In addition, as has also been previously mentioned, the outlet of the tube is fixed in a set position in the cross-section of the duct, which in turn fixes the position of instruments situated at the tube outlet. This significantly improves the effect of the laser treatment since, with current techniques, the laser can oscillate back and forth within the space during the treatment. These movements can depend on movements of the patient's internal organs or that the operator has difficulty in holding the instrument in position.

Even if the arrangement according to the invention has been described in connection to the treatment of bile duct stones, it can be used with advantage for other measures such as, for example, taking tissue samples, internal ultrasound, etc.

The arrangement can also be used with advantage for other procedures where an instrument has to be introduced through sensitive ducts or channels in the body. When applicable, the dimensions of the arrangement should be adapted to the actual task.

## Claims

1. Arrangement for treating stones in bile ducts intended to be inserted via a dilatation catheter through body tissue into a bile duct for the localisation and later removal of stones, as well as intended to constitute a channel for the subsequent insertion of instruments that protects the walls of the ducts, the arrangement comprising a flexible tube section (1) with an internal axial main channel extending from a front end to a rear end and an inflatable balloon device (3) that can be regulated in a radial direction being arranged directly adjacent to the front end of the tube section,
**characterised in that** said balloon device (3) is arranged asymmetrically around the front end of the tube section, and that the balloon device in its initial starting position has essentially the same external cross-sectional shape as the immediately adjacent tube section (1).

2. Arrangement according to claim 1 **characterised in that** the rear end has an inlet for inserting instruments through the main channel and that the inlet has an elasticised sealing device (2) that seals tightly around the inserted instrument.

3. Arrangement according to claim 1 or 2 **characterised in that** the balloon device (3) is connected to a pressure-regulating device via a channel (4) in the tube's casing.

4. Arrangement according to any of claims 1-3 **characterised in that** a marker of a material that resists X-ray beams is arranged in a specified position adjacent to the front end.

5. Arrangement according to any of the previous claims **characterised in that** a valve device (5) is arranged at the rear end of the tube for regulating the opening of a liquid pathway to the main channel.

6. Arrangement according to any of the previous claims **characterised in that** the balloon device is divided into sections in a peripheral direction and that the sections can be manoeuvred individually by means of channels connected to a pressure-regulating device.

## Patentansprüche

1. Anordnung zur Behandlung von Steinen in den Gallengängen, welche zur Lokalisierung und späteren Entfernung von Steinen über ein Dilatationskatheter durch das Körpergewebe in einen Gallengang einzuführen ist und einen Kanal zur nachfolgenden Einführung von Instrumenten bildet, der die Wände der Gänge schützt, wobei die Anordnung ein flexibles Rohrstück (1) mit einem inneren sich von einem Vorderende zu einem Hinterende erstreckenden axialen Hauptkanal und einer aufblasbaren Ballonvorrichtung (3) aufweist, welche in radialer Richtung regelbar ist und direkt am vorderen Ende des Rohrstücks anschliesst, **dadurch gekennzeichnet, dass** die Ballonvorrichtung (3) asymmetrisch um das Vorderende des Rohrstücks angeordnet ist, und dass die Ballonvorrichtung in der anfänglichen Ausgangslage im wesentlichen die gleiche äussere Querschnittsform aufweist wie das unmittelbar anschliessende Rohrstück (1).

2. Anordnung nach Anspruch 1, **dadurch gekennzeichnet, dass** das Hinterende einen Einlass zum Einführen von Instrumenten durch den Hauptkanal aufweist, und dass der Einlass eine elastische Dichtvorrichtung (2) aufweist, welche das eingeführte Instrument dicht umschliesst.

3. Anordnung nach Anspruch 1 oder 2, **dadurch gekennzeichnet, dass** die Ballonvorrichtung (3) über einen Kanal (4) im Mantel des Rohrs mit einem Druckregler verbunden ist.

4. Anordnung nach einem der Ansprüche 1-3, **dadurch gekennzeichnet, dass** an einer bestimmten Stelle beim Vorderende eine Markierung aus einem röntgenfesten Material angebracht ist.

5. Anordnung nach einem der vorangehenden Ansprüche, **dadurch gekennzeichnet, dass** beim Hinterende des Rohrs eine Ventilvorrichtung (5) zur Regelung der Öffnung einer Flüssigkeitsleitung zum Hauptkanal angeordnet ist.

6. Anordnung nach einem der vorangehenden Ansprüche, **dadurch gekennzeichnet, dass** die Ballonvorrichtung in Umfangsrichtung in Abschnitte unterteilt ist, und dass die Abschnitte über Kanäle, die mit einem Druckregler verbunden sind, einzeln bedienbar sind.

## Revendications

1. Arrangement pour le traitement de calculs dans les conduits biliaires, destiné à être introduit dans un conduit biliaire à travers le tissu corporel par l'intermédiaire d'un cathéter de dilatation pour la localisation et l'élimination ultérieure de calculs, et formant un canal pour l'introduction subséquente d'instruments qui protège les parois des conduits, ledit arrangement comprenant un élément tubulaire flexible (1) avec un canal interne axial principal qui s'étend de l'extrémité avant jusqu'à l'extrémité arrière, ainsi qu'un dispositif de ballonnet (3) gonfable qui est réglable en direction radiale, directement adjacent à l'extrémité avant de l'élément tubulaire, **caractérisé en ce que** ledit dispositif de ballonnet (3) est agencé de manière asymétrique autour de l'extrémité avant de l'élément tubulaire, et **en ce que** le dispositif de ballonnet présente, dans sa position de départ initiale, une coupe transversale de la même forme essentiellement que l'élément tubulaire (1) immédiatement adjacent.

2. Arrangement selon la revendication 1, **caractérisé en ce que** l'extrémité arrière comporte une entrée pour l'introduction d'instruments à travers le canal principal, et **en ce que** l'entrée est munie d'un dispositif d'étanchéité (2) élastique qui entoure de manière étanche l'instrument introduit.

3. Arrangement selon la revendication 1 ou 2, **caractérisé en ce que** le dispositif de ballonnet (3) est relié à un dispositif régulateur de pression par l'intermédiaire d'un canal (4) dans l'enveloppe du tube.

4. Arrangement selon l'une quelconque des revendications 1-3, **caractérisé en ce qu'**un marquage d'un matériau résistant aux rayons X est pourvu à un emplacement défini à proximité de l'extrémité avant.

5. Arrangement selon l'une quelconque des revendications précédentes, **caractérisé en ce qu'**un dispositif de soupape (5) est agencé à l'extrémité arrière du tube pour régler l'ouverture d'une amenée de liquide au canal principal.

6. Arrangement selon l'une quelconque des revendications précédentes, **caractérisé en ce que** le dispositif de ballonnet est divisé en sections en direction périphérique, et **en ce que** les sections peuvent être actionnées individuellement au moyen de canaux reliés à un dispositif régulateur de pression.
